(19) Europäisches Patentamt
European Patent Office
Office européen des brevets

(11) **EP 3 878 460 A1**

(12) **EUROPEAN PATENT APPLICATION**
published in accordance with Art. 153(4) EPC

(43) Date of publication:
**15.09.2021 Bulletin 2021/37**

(51) Int Cl.:
*A61K 36/82* (2006.01)   *A23L 33/00* (2016.01)
*A23L 5/20* (2016.01)

(21) Application number: **19881736.3**

(22) Date of filing: **04.11.2019**

(86) International application number:
**PCT/KR2019/014810**

(87) International publication number:
**WO 2020/096299 (14.05.2020 Gazette 2020/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **05.11.2018 KR 20180134620**
**01.04.2019 KR 20190037810**
**17.10.2019 KR 20190129034**

(71) Applicant: **Amorepacific Corporation**
**Seoul 04386 (KR)**

(72) Inventors:
• **KIM, Ayoung**
**Yongin-Si, Gyeonggi-do 17074 (KR)**
• **KIM, Hyungsu**
**Yongin-Si, Gyeonggi-do 17074 (KR)**
• **PARK, Wonseok**
**Yongin-Si, Gyeonggi-do 17074 (KR)**
• **CHO, Si Young**
**Yongin-Si, Gyeonggi-do 17074 (KR)**
• **HONG, Yong Deog**
**Yongin-Si, Gyeonggi-do 17074 (KR)**
• **KWON, Gusang**
**Yongin-Si, Gyeonggi-do 17074 (KR)**

(74) Representative: **Germain Maureau**
**12, rue Boileau**
**69006 Lyon (FR)**

(54) **GREEN TEA EXTRACT HAVING MODIFIED CONSTITUENT CONTENT AND COMPOSITION COMPRISING SAME**

(57) The present invention discloses a green tea extract having modified constituent content, which exhibits reduced hepatotoxicity, and a composition comprising same as an active ingredient for enhancing blood circulation, inhibiting lipid accumulation in hepatocytes, inhibiting gingivalis bacteria, or increasing cerebral blood flow. Specifically, a green tea extract according to an aspect of the present invention has constituents that are modified by treatment at a high temperature to thus have lower concentration of that which causes hepatotoxicity than conventional green tea extract, thereby allowing hepatocyte damage to be reduced. The composition comprising such a green tea extract according to the present invention is of high safety even when being ingested in large amounts and exhibits excellent efficacy in enhancing blood circulation, inhibiting lipid accumulation in hepatocytes, inhibiting gingivalis bacteria, or increasing cerebral blood flow.

[FIG. 2]

## Description

**[Technical Field]**

**Cross-Reference to Related Application**

This application claims the priority of Korean Patent Application No. 10-2018-0134620 filed on November 5, 2018, Korean Patent Application No. 10-2019-0037810 filed on April, 1, 2019 and Korean Patent Application No. 10-2019-0129034 filed on October 17, 2019, the disclosure of which is incorporated herein by reference in its entirety.

**Technical Field**

The present disclosure relates to a green tea extract having modified contents of ingredients, which exhibits reduced hepatotoxicity, and a composition containing the same as an active ingredient for enhancing blood circulation, inhibiting lipid accumulation in hepatocytes, inhibiting gingivalis bacteria or increasing cerebral blood flow.

**[Background Art]**

The major causes of fatty liver are chronic drinking, obesity caused by overeating and diabetes. In addition, fatty liver may occur when drugs with strong toxicity are taken, antibiotics are misused during pregnancy or aspirin is abused to alleviate fever in children with febrile infectious diseases. Fatty liver is recovered easily through regeneration of hepatocytes under good physical conditions including immune conditions. But, otherwise, the function of hepatocytes declines as fat is accumulated in the hepatocytes and important constituents of the cells including nuclei are pushed to the periphery of the cells. In addition, the fat accumulated in the hepatocytes compress microvessels and lymph glands between the hepatocytes, causing damage to the circulation of blood and lymph in the liver. As a result, the necessary oxygen and nutrients cannot be delivered to the hepatocytes and the liver function declines.

In normal blood vessels, homeostasis is maintained as the activation and inhibition of the hemostasis mechanism are balanced. But, in blood circulation diseases, thrombosis may be caused by excessive hemostatic activation or blood clot formation, which interrupts blood flow. In normal vascular endothelial cells, inhibitory factors such as nitric oxide (NO) and nitric oxide (PGI2) are released, which protect the blood vessel wall and inhibit platelet adhesion and activation. However, platelet coagulation is promoted if the endothelial cells are damaged. Thrombosis collectively refers to the diseases caused by the blood clots that block blood vessels.

Periodontitis has various causes and symptoms. One of the main causes of periodontitis is the microorganisms in the mouth. They produce insoluble glucans using sugars, which form dental plaques by adhering to tooth surfaces together with various micro-colonies. The dental plaque causes tooth decay and periodontitis. Therefore, the most effective method for preventing periodontitis may be to use a substance which inhibits the activity of the microorganisms and facilitates blood flow to the gum where inflammation has occurred. The microorganisms distributed in the mouth cause bad breath, periodontal disease, etc. Some of the microorganisms are known as opportunistic pathogens which have the capacity of causing diseases. Several species of pathogenic microorganisms which reside in the mouth and cause periodontitis are known. Among them, *Prevotella intermedia, Porphyromonas gingivalis,* etc. are known as the main pathogens responsible for periodontitis.

The brain performs its functions by receiving blood pumped out of the heart and obtaining oxygen or nutrients such as glucose, etc. therefrom. Because the brain lacks the ability to store oxygen and glucose in tissues, unlike other bodily tissues, disorder of the blood flow to the brain due to the narrowing or blockage of the cerebral blood vessels by arteriosclerosis, thrombosis, etc. may cause brain diseases including nerve cell damage owing to oxygen deficiency and lack of glucose. The diseases directly or indirectly associated with the disorder of cerebral blood flow include anemia, stroke, vascular dementia, Parkinsonism, Huntington's disease, etc.

Meanwhile, green tea is widely used as a food supplement for improving obesity or metabolic disease because it is effective in reducing body weight and lowering cholesterol level. However, with the recent report that the intake of a large quantity of green tea extract can cause liver toxicity, the safety issue of the green tea extract is emerging. Accordingly, the discovery of a composition of the green tea extract with reduced liver injury is necessary.

**[Disclosure]**

**[Technical Problem]**

The present disclosure is directed to providing a green tea extract with reduced hepatotoxicity, specifically hepatocyte damage, and a composition containing the same as an active ingredient and having high safety for enhancing blood circulation, inhibiting lipid accumulation in hepatocytes, inhibiting gingivalis bacteria or increasing cerebral blood flow.

[Technical Solution]

In an aspect, the present disclosure provides a green tea extract with reduced hepatotoxicity, which contains 8 wt% or less of epigallocatechin (EGC) based on the total weight of the extract.

In another aspect, the present disclosure provides a composition for enhancing blood circulation, which contains a green tea extract with reduced hepatotoxicity, containing 8 wt% or less of epigallocatechin (EGC) based on the total weight of the extract, as an active ingredient.

In another aspect, the present disclosure provides a composition for inhibiting lipid accumulation in hepatocytes, which contains a green tea extract with reduced hepatotoxicity, containing 8 wt% or less of epigallocatechin (EGC) based on the total weight of the extract, as an active ingredient.

In another aspect, the present disclosure provides a composition for inhibiting gingivalis bacteria, which contains a green tea extract with reduced hepatotoxicity, containing 8 wt% or less of epigallocatechin (EGC) based on the total weight of the extract, as an active ingredient.

In another aspect, the present disclosure provides a composition for increasing cerebral blood flow, which contains a green tea extract with reduced hepatotoxicity, containing 8 wt% or less of epigallocatechin (EGC) based on the total weight of the extract, as an active ingredient.

[Advantageous Effects]

A green tea extract according to an aspect of the present disclosure, which has modified contents of ingredients through treatment at high temperature, contains the ingredients that cause hepatotoxicity at lower concentrations as compared to the existing green tea extract. Therefore, hepatocyte damage by the existing green tea extract can be reduced effectively and safety can be ensured even when it is ingested in large amounts.

A composition according to another aspect of the present disclosure, which contains the green tea extract as an active ingredient, exhibits an excellent effect of preventing or treating a thrombotic disease as well as high safety due to superior effect of enhancing blood circulation.

A composition according to another aspect of the present disclosure, which contains the green tea extract as an active ingredient, exhibits an excellent effect of preventing or treating fatty liver as well as high safety due to superior effect of inhibiting lipid accumulation in hepatocytes.

A composition according to another aspect of the present disclosure, which contains the green tea extract as an active ingredient, exhibits an excellent effect of preventing or treating periodontitis as well as high safety due to superior effect of improving *Porphyromonas gingivalis.*

A composition according to another aspect of the present disclosure, which contains the green tea extract as an active ingredient, exhibits an excellent effect of improving cognitive function as well as high safety due to superior effect of increasing cerebral blood flow.

[Brief Description of Drawings]

FIG. 1 shows the chromatogram of a common green tea extract of Example 1 (sample 1).

FIG. 2 shows the chromatogram of a high-temperature-processed green tea extract according to an aspect of the present disclosure (sample 2).

FIG. 3 shows a result of investigating the hepacytotoxicity of a high-temperature-processed green tea extract in Test Example 1.

FIG. 4 shows a result of investigating the effect of a high-temperature-processed green tea extract on hepatocyte damage in Test Example 2.

FIG. 5 shows a result of investigating the effect of three catechins EGCG, GCG and EGC on hepatocyte damage in Test Example 4.

FIG. 6 shows a result of evaluating the blood circulation-enhancing effect of a high-temperature-processed green tea extract in Test Example 5.

FIG. 7 shows a result of evaluating the effect of inhibiting lipid accumulation in hepatocytes of a high-temperature-processed green tea extract in Test Example 6.

FIG. 8 shows a result of evaluating the effect of inhibiting lipid accumulation in hepatocytes of GCG and EGCG in Test Example 7.

FIG. 9 shows a result of evaluating the effect of increasing cerebral blood flow of a high-temperature-processed green tea extract in Test Example 9.

FIG. 10 shows a result of evaluating the effect of increasing cerebral blood flow of a high-temperature-processed green tea extract in Test Example 9.

**[Best Mode]**

In the present disclosure, a "green tea extract" includes an extract obtained from the evergreen shrub tea *(Camellia sinensis),* which belongs to the family *Theaceae,* or from the leaves of tea fermented by inoculating with *Bacillus subtilis,* regardless of extraction method, extraction solvent, extracted ingredients or extract type, as well as a fraction fractionated from the extract using a specific solvent. The tea may refer to one or more selected from a group consisting of the leaf, flower, stem, fruit, root and duramen of tea tree. Specifically, it may refer to leaf. In addition, the extract may be specifically in powder from. The extraction or fractionation may be performed using water, an organic solvent or a mixture solvent thereof. As the organic solvent, an alcohol such as isopropanol, acetone, hexane, ethyl acetate, carbon dioxide or a mixture solvent thereof may be used, although not being limited thereto. The extraction or fractionation may be performed at room temperature or elevated temperatures under a condition where the destruction of the active ingredients of green tea is prevented or minimized. The alcohol may be a $C_1$-$C_5$ lower alcohol. The number and method of the extraction or fractionation is not particularly limited. For example, such methods as cryoprecipitation extraction, ultrasonic extraction, reflux condensation extraction, hot water extraction, etc. may be used. Specifically, after extracting or fractionating active ingredients by cryoprecipitation or heating and, followed by filtration, the green tea extract of the present disclosure may be obtained by concentrating the filtrate under reduced pressure.

In the present disclosure "epicatechin" includes epigallocatechin (EGC), (-)-epicatechin (EC), (-)-epigallocatechin gallate (EGCG) and epicatechin 3-O-gallate (ECG).

In the present disclosure an "epicatechin epimer" includes gallocatechin (GC), catechin (C), (-)-gallocatechin gallate (GCG) and catechin gallate (CG).

In an aspect, the present disclosure may relate to a green tea extract with reduced hepatotoxicity, which contains 8 wt% or less of epigallocatechin (EGC) based on the total weight of the extract.

In another aspect, the present disclosure may relate to a method for reducing hepatotoxicity, which includes a step of administering an effective amount of a green tea extract with reduced hepatotoxicity, containing 8 wt% or less of epigallocatechin (EGC) based on the total weight of the extract, to a subject in need thereof.

In another aspect, the present disclosure may relate to a use of a green tea extract with reduced hepatotoxicity, containing 8 wt% or less of epigallocatechin (EGC) based on the total weight of the extract, for preparation of a composition reducing hepatotoxicity.

In another aspect, the present disclosure may relate to a green tea extract with reduced hepatotoxicity, containing 8 wt% or less of epigallocatechin (EGC) based on the total weight of the extract, for use in reducing hepatotoxicity.

In another aspect, the present disclosure may relate to a composition for enhancing blood circulation, which contains the green tea extract as an active ingredient.

In another aspect, the composition may increase nitric oxide (NO) in vascular endothelial cells, and may exhibit blood circulation-enhancing effect by increasing nitric oxide in vascular endothelial cells.

In another aspect, the present disclosure may relate to a method for enhancing blood circulation, which includes a step of administering an effective amount of a green tea extract with reduced hepatotoxicity, containing 8 wt% or less of epigallocatechin (EGC) based on the total weight of the extract, to a subject in need thereof.

In another aspect, the present disclosure may relate to a use of a green tea extract with reduced hepatotoxicity, containing 8 wt% or less of epigallocatechin (EGC) based on the total weight of the extract, for preparing a composition for enhancing blood circulation.

In another aspect, the present disclosure may relate to a green tea extract with reduced hepatotoxicity, containing 8 wt% or less of epigallocatechin (EGC) based on the total weight of the extract, for use in enhancing blood circulation.

In another aspect, the present disclosure may relate to a composition for inhibiting lipid accumulation in hepatocytes, which contains the green tea extract as an active ingredient.

In another aspect, the present disclosure may relate to a method for inhibiting lipid accumulation in hepatocytes, which includes a step of administering an effective amount of a green tea extract with reduced hepatotoxicity, containing 8 wt% or less of epigallocatechin (EGC) based on the total weight of the extract, to a subject in need thereof.

In another aspect, the present disclosure may relate to a use of a green tea extract with reduced hepatotoxicity, containing 8 wt% or less of epigallocatechin (EGC) based on the total weight of the extract, for preparing a composition for inhibiting lipid accumulation in hepatocytes.

In another aspect, the present disclosure may relate to a green tea extract with reduced hepatotoxicity, containing 8 wt% or less of epigallocatechin (EGC) based on the total weight of the extract, for use in inhibiting lipid accumulation in hepatocytes.

In another aspect, the present disclosure may relate to a composition for inhibiting gingivalis bacteria, which contains the green tea extract as an active ingredient.

In another aspect, the present disclosure may relate to a method for inhibiting gingivalis bacteria, which includes a step of administering an effective amount of a green tea extract with reduced hepatotoxicity, containing 8 wt% or less of epigallocatechin (EGC) based on the total weight of the extract, to a subject in need thereof.

In another aspect, the present disclosure may relate to a use of a green tea extract with reduced hepatotoxicity, containing 8 wt% or less of epigallocatechin (EGC) based on the total weight of the extract, for preparing a composition for inhibiting gingivalis bacteria.

In another aspect, the present disclosure may relate to a green tea extract with reduced hepatotoxicity, containing 8 wt% or less of epigallocatechin (EGC) based on the total weight of the extract, for use in inhibiting gingivalis bacteria.

In another aspect, the gingivalis bacteria may be *Porphyromonas gingivalis.*

In another aspect, the present disclosure may relate to a composition for increasing cerebral blood flow, which contains the green tea extract as an active ingredient. In another aspect, the composition may increase the amount of oxygen in cerebral blood flow and may, specifically, increase blood flow in the frontal lobe of the brain.

In another aspect, the present disclosure may relate to a method for increasing cerebral blood flow, which includes a step of administering an effective amount of a green tea extract with reduced hepatotoxicity, containing 8 wt% or less of epigallocatechin (EGC) based on the total weight of the extract, to a subject in need thereof.

In another aspect, the present disclosure may relate to a use of a green tea extract with reduced hepatotoxicity, containing 8 wt% or less of epigallocatechin (EGC) based on the total weight of the extract, for preparing a composition for increasing cerebral blood flow.

In another aspect, the present disclosure may relate to a green tea extract with reduced hepatotoxicity, containing 8 wt% or less of epigallocatechin (EGC) based on the total weight of the extract, for preparing a composition for use in increasing cerebral blood flow.

In an aspect of the present disclosure, the administration may be made according to the administration method and administration dosage described herein.

In an aspect, the EGC may be contained in an amount of 8 wt% or less, 7 wt% or less, 6 wt% or less, 5 wt% or less, 4.7 wt% or less, 4.56 wt% or less, 4.3 wt% or less, 4 wt% or less, 3.53 wt% or less, 3.5 wt% or less, 3 wt% or less, 2 wt% or less, 1 wt% or less, 0.8 wt% or less, 0.5 wt% or less, 0.3 wt% or less or 0.1 wt% or less based on the total weight of the extract. In another aspect, the EGC may be contained in an amount of 0.0001 wt% or more, 0.001 wt% or more, 0.01 wt% or more, 1 wt% or more, 2 wt% or more, 3 wt% or more, 3.5 wt% or more, 3.53 wt% or more, 4 wt% or more, 4.3 wt% or more, 4.56 wt% or more, 4.7 wt% or more, 5 wt% or more, 6 wt% or more or 7 wt% or more based on the total weight of the extract. When the EGC is contained within the above-described content ranges, a superior hepatotoxicity-reducing effect may be achieved.

In an aspect, the extract may contain 4-15 wt% of (-)-gallocatechin gallate (GCG) and 4-15 wt% of (-)-epigallocatechin gallate (EGCG) based on the total weight of the extract.

The GCG may be contained in an amount of 4 wt% or more, 5 wt% or more, 5.3 wt% or more, 5.59 wt% or more, 5.7 wt% or more, 6 wt% or more, 7 wt% or more, 8 wt% or more, 9 wt% or more, 10 wt% or more, 11 wt% or more, 12 wt% or more, 13 wt% or more or 14 wt% or more based on the total weight of the extract. In another aspect, the GCG may be contained in an amount of 15 wt% or less, 14 wt% or less, 13 wt% or less, 12 wt% or less, 11 wt% or less, 10 wt% or less, 9 wt% or less, 8 wt% or less, 7 wt% or less, 6 wt% or less, 5.7 wt% or less, 5.59 wt% or less, 5.3 wt% or less or 5 wt% or less based on the total weight of the extract

In an aspect, the EGCG may be contained in an amount of 4 wt% or more, 5 wt% or more, 5.2 wt% or more, 5.27 wt% or more, 5.5 wt% or more, 6 wt% or more, 7wt% or more, 8wt% or more, 9 wt% or more, 10 wt% or more, 11 wt% or more, 12 wt% or more, 13 wt% or more or 14 wt% or more based on the total weight of the extract. In another aspect, the EGCG may be contained in an amount of 15 wt% or less, 14 wt% or less, 13 wt% or less, 12 wt% or less, 11 wt% or less, 10 wt% or less, 9 wt% or less, 8 wt% or less, 7 wt% or less, 6 wt% or less, 5.5 wt% or less, 5.27 wt% or less, 5.2 wt% or less or 5 wt% or less based on the total weight of the extract.

In another exemplary embodiment, the total content of GCG and EGCG in the extract may be 30 wt% or less based on the total weight of the extract. In an aspect, the total content of GCG and EGCG may be 30 wt% or less, 25 wt% or less, 20 wt% or less, 18 wt% or less, 16 wt% or less, 15 wt% or less, 14 wt% or less, 12 wt% or less, 11 wt% or less, 10.86 wt% or less, 10 wt% or less or 9 wt% or less based on the total weight of the extract. In another aspect, the total content of GCG and EGCG may be 8 wt% or more, 10 wt% or more, 10.86 wt% or more, 11 wt% or more, 12 wt% or more, 13 wt% or more, 14 wt% or more, 16 wt% or more, 18 wt% or more, 20 wt% or more or 25 wt% or more based on the total weight of the extract.

In another exemplary embodiment, the content of epicatechin in the extract may be 20 wt% or less based on the total weight of the extract. In an aspect, the epicatechin content may be 20 wt% or less, 18 wt% or less, 16 wt% or less, 15 wt% or less, 14 wt% or less, 12 wt% or less, 11.1 wt% or less or 10 wt% or less based on the total weight of the extract. In another aspect, the epicatechin content may be 9 wt% or more, 10 wt% or more, 11.1 wt% or more, 12 wt% or more, 13 wt% or more, 14 wt% or more, 16 wt% or more or 18 wt% or more based on the total weight of the extract.

In another exemplary embodiment, the total content of eight catechins in the extract, i.e., the total content of (-)-epigallocatechin gallate (EGCG), epigallocatechin (EGC) (-)epicatechin (EC), epicatechin 3-O-gallate (ECG), gallocatechin gallate (GCG), gallocatechin (GC), catechin (C) and catechin gallate (CG) in the extract, may be 19-30 wt% based on the total weight of the extract. In an aspect, the total content of the eight catechins may be 19 wt% or more, 21 wt% or more, 23 wt% or more, 24 wt% or more, 24.5 wt% or more, 25 wt% or more, 26 wt% or more, 27 wt% or more, 28 wt% or more or 29 wt% or more based on the total weight of the extract. In another aspect, the total content of eight catechins may be 30 wt% or less, 29 wt% or less, 28 wt% or less, 27 wt% or less, 26 wt% or less, 25 wt% or less, 24.5 wt% or less, 24 wt% or less, 23 wt% or less or 21 wt% or less based on the total weight of the extract.

In another exemplary embodiment, the extract may be an extract obtained by extracting one or more times with one or more of water and a $C_1$-$C_4$ alcohol. In an aspect, the alcohol may be ethanol. In another aspect, the alcohol may be 20% or higher, 30% or higher, 40% or higher, 50% or higher, 60% or higher or 70% or higher ethanol. In another aspect, the alcohol may be 70% or lower, 60% or lower, 50% or lower, 40% or lower or 30% or lower ethanol.

In an exemplary embodiment, the extract may be contained in a functional health food or pharmaceutical composition.

In an exemplary embodiment, the content of the green tea extract in the functional health food or pharmaceutical composition may be 1-100 wt% based on the total weight of the composition. In an aspect, the content of the extract in the composition may be 1 wt% or more, 10 wt% or more, 20 wt% or more, 30 wt% or more, 40 wt% or more, 50 wt% or more, 60 wt% or more, 70 wt% or more, 80 wt% or more or 90 wt% or more. In another aspect, the content of the extract in the composition may be 100 wt% or less, 90 wt% or less, 80 wt% or less, 70 wt% or less, 60 wt% or less, 50 wt% or less, 40 wt% or less, 30 wt% or less or 20 wt% or less.

In another exemplary embodiment, the administration dosage of the green tea extract may be 5-1000 mg/kg/day. In an aspect, the administration dosage may be 5 mg/kg/day or more, 100 mg/kg/day or more, 200 mg/kg/day or more, 300 mg/kg/day or more, 400 mg/kg/day or more, 500 mg/kg/day or more, 600 mg/kg/day or more, 700 mg/kg/day or more, 800 mg/kg/day or more or 900 mg/kg/day or more. In another aspect, the administration dosage may be 1000 mg/kg/day or less, 900 mg/kg/day or less, 800 mg/kg/day or less, 700 mg/kg/day or less, 600 mg/kg/day or less, 500 mg/kg/day or less, 400 mg/kg/day or less, 300 mg/kg/day or less, 200 mg/kg/day or less, 100 mg/kg/day or less, 50 mg/kg/day or less or 10 mg/kg/day or less.

The formulation of the food composition is not particularly limited. For example, the composition may be formulated into a tablet, a granule, a pill, a powder, a liquid such as a drink, a caramel, a gel, a bar, a tea bag, etc. Each formulation of the food composition may be prepared without difficulty by those of ordinary skill in the art by mixing the active ingredient with an ingredient in consideration of the formulation or purpose of use. A synergistic effect may be achieved when an additional ingredient is used. The food may also be a functional health food.

The composition may be administered by various methods such as simple ingestion, drinking, injection, spraying, squeezing, etc.

For the food composition according to an aspect of the present disclosure, determination of the administration dosage of the active ingredient is within the level of those of ordinary skill in the art and may vary depending on various factors such as the age and health condition of a subject, the presence of a complication, etc.

For example, the food composition according to an aspect of the present disclosure may be various foods such as chewing gum, caramel, candy, ices, confectionery, etc., beverages such as soft drinks, mineral water, alcoholic beverages, etc., or functional health food products such as vitamins, minerals, etc.

In addition, the food composition according to an aspect of the present disclosure may contain various nutrients, vitamins, minerals (electrolytes), flavorants such as synthetic flavorants or natural flavorants, colorants, extenders (cheese, chocolate, etc.), pectic acid and its salts, alginic acid and its salts, organic acids, protective colloidal thickeners, pH control agents, stabilizers, antiseptics, glycerin, alcohols, carbonating agents used in carbonated drinks, etc. In addition, the food composition according to an aspect of the present disclosure may contain a pulp for preparing natural fruit juice, fruit juice drinks and vegetable drinks. These ingredients may be used independently or in combination. Although the proportion of the additives is of no great importance, they are usually contained in a range of about 0-60 parts by weight per 100 parts by weight of the composition according to an aspect of the present disclosure.

The food composition according to an aspect of the present disclosure may be one with reduced hepatotoxicity of a green tea extract. More specifically, the food composition may be one with reduced hepatotoxicity by EGC of the green tea extract.

The pharmaceutical composition according to an aspect of the present disclosure may be administered orally or parenterally, e.g., rectally, topically, transdermally, intravenously, intramuscularly, intraperitoneally, subcutaneously, etc. A formulation for oral administration may be a tablet, a pill, a soft or hard capsule, a granule, a powder, a fine granule, a liquid, an emulsion or a pellet, although not being limited thereto. A formulation for parenteral administration may be a solution, a suspension, an emulsion, a gel, an injection, a medicinal drop, a suppository, a patch or a spray, although not being limited thereto. The formulation may be prepared easily according to common methods in the art and my further contain a surfactant, an excipient, a wetting agent, an emulsification accelerator, a suspending agent, a salt or buffer for control of osmotic pressure, a colorant, a flavor, a stabilizer, an antiseptic, a preservative or other commonly used adjuvants.

The composition according to an aspect of the present disclosure may also contain a pharmaceutically acceptable salt. The salt may include: (1) an acid addition salt formed from an inorganic acid such as hydrochloric acid, hydrobromic acid, sulfuric acid, nitric acid, phosphoric acid; or formed from an organic acid such as acetic acid, propionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, ethane-1,2-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid or muconic acid; or (2) a salt formed when an acidic proton present in the parent compound is replaced.

The application amount or administration dosage of the pharmaceutical composition according to an aspect of the present disclosure will vary depending on the age, sex and body weight of a subject, pathological condition and severity thereof, administration route or the discretion of a diagnoser. Determination of the administration dosage of the active ingredient based on these factors is within the level of those of ordinary skill in the art.

Hereinafter, the present disclosure is described in more detail through examples and test examples. However, those examples are provided only for the understanding of the present disclosure and the scope of the present disclosure is not limited by them.

### [Example 1] Preparation of common green tea extract and high-temperature-processed green tea extract

After adding 1000 mL of 50% ethanol to 100 g of green tea *(Camellia sinensis,* Jeju O' Sulloc Farm), the mixture was refluxed at 60 °C for 1 hour under stirring. After cooling to room temperature and filtering, 23 g of a notified green tea extract (GT-LE-35CAT, sample 1) was obtained as deep brown powder by distilling the obtained solution under reduced pressure (yield: 23%).

Meanwhile, for preparation of a high-temperature-processed green tea extract, 1000 mL of 50% ethanol was added to 100 g of green tea *(Camellia sinensis,* Jeju O'Sulloc Farm) and the mixture was refluxed at 60 °C for 1 hour under stirring. After concentration, the resulting solution was stirred for 1-7 hours under steam flow at 1.5 kgf/cm$^2$. After cooling to room temperature and filtering insoluble materials, 10 g of a high-temperature-processed green tea extract was obtained by concentrating the solution under reduced pressure. For high-temperature-processed green tea extracts obtained at different stirring times (1-7 hours), the change in the content of eight catechins was measured using an apparatus described in Table 1. It was confirmed that the conversion of EGCG to GCG by heat was highest at 5 hours and the amount of the eight catechins did not decrease any more. 10 g of the obtained high-temperature-processed green tea extract (HTP-GTE) was designated as sample 2.

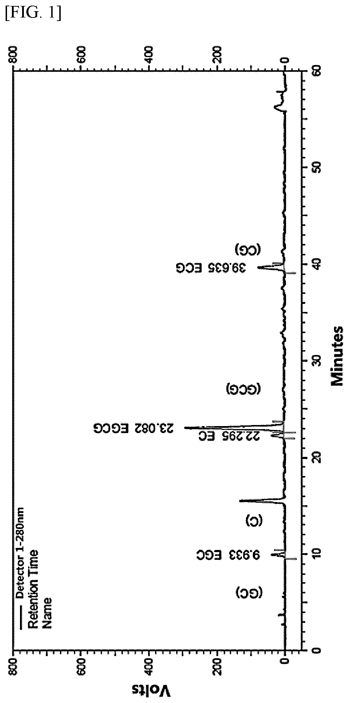

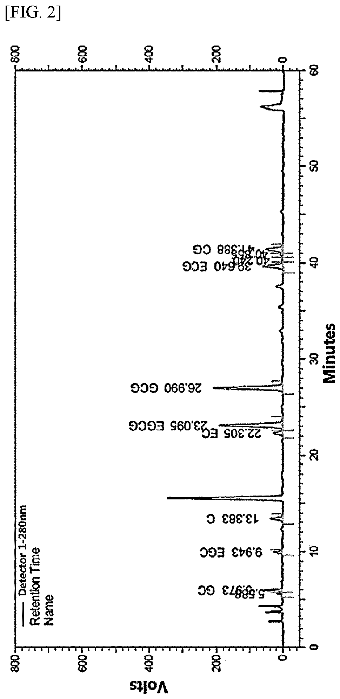

A result of analyzing the composition of the two obtained extracts and the analysis condition are shown in Table 1 (composition analysis condition for samples 1 and 2), Table 2 (composition analysis result for sample 1) and Table 3 (composition analysis result for sample 2). In addition, the chromatograms of the two extracts are shown in FIG. 1 (sample 1) and FIG. 2 (sample 2). It was confirmed that the sample 2 has a composition different from that of the existing green tea extract. Specifically, the sample 2 had significantly lower contents of EGC (3.53 wt%), EGCG (5.27 wt%) and total catechins (24.41 wt%) as compared to the sample 1, but also contained four epicatechin epimers not found in the sample 1.

[Table 1]

| | |
|---|---|
| Column | Thermo Fisher C18 5 $\mu$m, 4.6 $\times$ 250 mm |
| Detector | UV 280 nm |
| Dilution | Gradient<br>A = 0.1% TFA (trifluoroacetic acid) in water<br>B = acetonitrile |
| Gradient profile | 0 min A (90) : B (10)<br>30 min A (85) : B (15)<br>42 min A (80) : B (20)<br>44 min A (5) : B (95)<br>49 min A (90) : B (10) |

(continued)

| Flow rate | 1 mL/min |
|---|---|
| Injection volume | 20 $\mu$L |

[Table 2]

| | GC | EGC | Caffeine | C | EC | GCG | EGCG | CG | ECG | Total epicatechins | Total epicatechin epimers | Total catechins |
|---|---|---|---|---|---|---|---|---|---|---|---|---|
| Sample 1 | 0 | 9.16 | 3.21 | 0 | 3.63 | 0 | 20.93 | 0 | 2.62 | 36.34 | 0 | 36.34 |

[Table 3]

| Sample 2 | GC | EGC | Caffeine | C | EC | GCG | EGCG | CG | ECG | Total epicatechins | Total epicatechin epimers | Total catechins |
|---|---|---|---|---|---|---|---|---|---|---|---|---|
| | 4.9 | 3.53 | 3.9 | 1.57 | 0.95 | 5.59 | 5.27 | 1.3 | 1.3 | 11.05 | 13.36 | 24.41 |

(In Table 2 and Table 3, GC: gallocatechin, EGC: epigallocatechin, C: catechin, EC: (-)epicatechin, GCG: gallocatechin gallate, EGCG: epigallocatechin gallate, CG: catechin gallate, ECG: epicatechin 3-O-gallate.)

(All the units in Table 2 and Table 3 are wt% of the corresponding ingredients based on the total weight of the green tea extract (samples 1 and 2).)

**[Test Example 1] Investigation of hepacytotoxicity of high-temperature-processed green tea extract**

In order to investigate the hepatotoxicity of the high-temperature-processed green tea extract obtained in Example 1 (sample 2), hepatocytes (HepG2 cells) acquired from Korean Cell Line Bank were seeded onto a 96-well plate (Falcon) with $1 \times 10^5$ cells per well and cultured for 24 hours in a 5% $CO_2$ incubator at 37 °C. Then, after treating with the sample 1 or sample 2 at 5, 10, 25, 50 or 100 μg/mL and culturing further for 24 hours, the medium was removed and cell viability was investigated using a CellTiter 96 AQueous One Solution cell proliferation assay kit (MTS, Promega Co. Madison, WI, USA). After treating the cells with 20 μL of a CellTiter 96 AQueous One Solution cell proliferation assay kit (MTS, Promega Co. Madison, WI, USA) solution added to 100 μL of RPMI1640 (Lonza), the number of live cells was quantified by measuring absorbance at 490 nm. The cell number or cell viability (%) was calculated by the following equation.

$$\text{Cell viability (\%)} = (\text{absorbance of sample-treated group - absorbance of reagent alone})$$

$$/ \text{ (absorbance of non-treated group - absorbance of reagent alone) x 100}$$

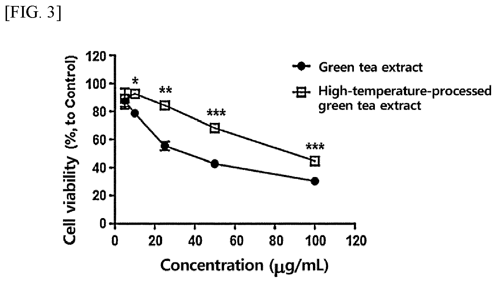

The result is shown in FIG. 3. It was confirmed that the sample 2 according to the present disclosure exhibits lower hepacytotoxicity than the sample 1 which is a common green tea extract.

**[Test Example 2] Investigation of effect of high-temperature-processed green tea extract on hepatocyte damage**

In order to investigate the effect of the high-temperature-processed green tea extract obtained in Example 1 (sample 2) on hepatocyte damage, release of enzymes from hepatocytes due to hepatocyte damage was analyzed using the MILLIPLEX Analyst software (Millipore). The experimental condition was the same as in Test Example 1. After treating the cells with the sample 1 or sample 2, the levels of malate dehydrogenase 1 (MDH 1), α-glutathione S-transferase (GSTα) and sorbitol dehydrogenase (SDH) in the cell culture were measured 24 later.

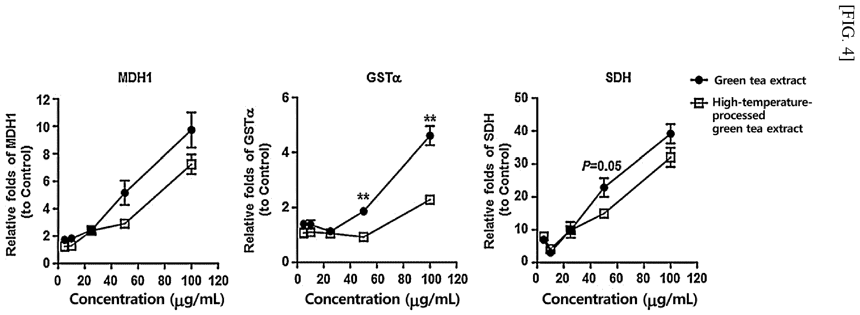

The result is shown in FIG. 4. It was confirmed that the levels of the three enzymes in the cell culture were lower when the cells were treated with the high-temperature-processed green tea extract (sample 2) as compared to when they were treated with the common green tea extract (sample 1).

That is to say, it was confirmed that the green tea extract sample 2 according to the present disclosure has smaller effect on hepatocyte damage as compared to the common green tea extract.

**[Test Example 3] Investigation of hepacytotoxicity of eight catechins**

In order to investigate the hepatotoxicity by eight catechins (EGCG, GCG, EGC, GC, ECG, CG, EC, C) present in the common green tea extract or the high-temperature-processed green tea extract, hepatocytes (HepG2 cells) acquired from Korean Cell Line Bank were seeded onto a 96-well plate (Falcon) with $1 \times 10^5$ cells per well and cultured for 24 hours in a 5% $CO_2$ incubator at 37 °C. Then, after treating with each of eight catechins (EGCG, GCG, EGC, GC, ECG, CG, EC, C) at 10, 50, 100 or 200 μM and culturing further for 24 hours, the medium was removed and cell viability was investigated using a CellTiter 96 AQueous One Solution cell proliferation assay kit (MTS, Promega Co. Madison, WI, USA). After treating the cells with 20 μL of a CellTiter 96 AQueous One Solution cell proliferation assay kit (MTS, Promega Co. Madison, WI, USA) solution added to 100 μL of RPMI1640 (Lonza), the number of live cells was quantified by measuring absorbance at 490 nm. The cell number or cell viability (%) was calculated as described in Test Example 1. $LD_{50}$ (lethal dose 50) values depending on concentrations were calculated based on the cell viability, and the result is given in Table 4.

[Table 4]

|  | EGCG | GCG | EGC | GC | ECG | CG | EC | C |
|---|---|---|---|---|---|---|---|---|
| $LD_{50}$ (μM) | 355.5 | 212.7 | 183.9 | 236.2 | 209.0 | 260.8 | 505.0 | 569.0 |

As seen from Table 4, EGC showed the lowest $LD_{50}$ value among the eight catechins. Accordingly, it was confirmed that EGC has the highest hepacytotoxicity.

**[Test Example 4] Investigation of effect of three catechins on hepatocyte damage**

In order to investigate the effect of the EGCG, GCG and EGC among the eight catechins on hepatocyte damage, release of enzymes from hepatocytes due to hepatocyte damage was analyzed using the MILLIPLEX Analyst software (Millipore). The experimental condition was the same as in Test Example 3. After treating the cells with EGCG, GCG or EGC, the levels of malate dehydrogenase 1 (MDH 1), $\alpha$-glutathione S-transferase (GST$\alpha$) and sorbitol dehydrogenase (SDH) in the cell culture were measured 24 later.

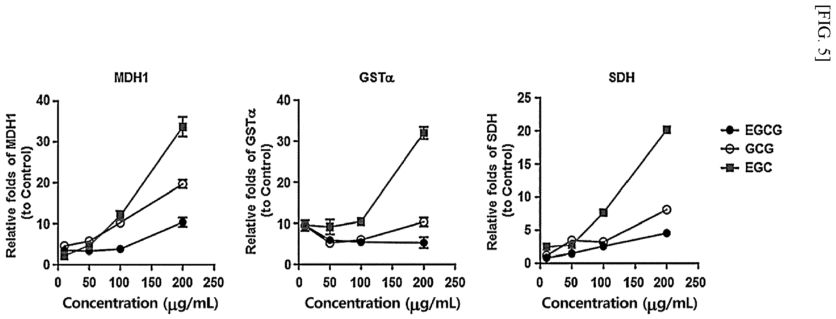

The result is shown in FIG. 5. It was confirmed that the levels of the three enzymes in the cell culture were the highest in the cell culture treated EGC as compared to the cell cultures treated with EGCG or GCG.

That is to say, it was confirmed that EGC has the largest effect on hepatocyte damage as compared to EGCG and GCG.

**[Test Example 5] Evaluation of blood circulation-enhancing effect of high-temperature-processed green tea extract**

Endothelial nitric oxide synthase (eNOS) is present in human vascular endothelial cells. When its activity is increased, blood vessels are dilated and blood circulation is promoted as nitric oxide (NO) is produced. In order to evaluate the blood circulation-enhancing effect of the green tea extract sample 1 and sample 2 of Example 1, human umbilical vein endothelial cells (HUVECs) were treated with each sample and the amount of produced NO was compared.

Specifically, vascular endothelial cells were seeded onto a clear-bottom, black-wall, 96-well plate (Falcon) with $2 \times 10^4$ cells per well and cultured in a 5% $CO_2$ incubator at 37 °C for 24 hours. After starving the cells for 4 hours with a fetal bovine serum (FBS)-depleted phenol red-free medium, the cells were washed twice with phosphate-buffered saline (PBS) after removing the medium. Then, after treating with 100 μL of 5 μM DAF-2DA dissolved in Hank's balanced salt solution (HBSS), the cells were cultured in a 5% $CO_2$ incubator at 37 °C for 20 minutes with light blocked using a foil. After measuring fluorescence before sample treatment (excitation: 485 nM, emission: 535 nM), fluorescence was measured with a microplate reader (Tecan, Salzburg, Austria) after treating each well with 100 μL of the sample 1 or the sample 2 at 6, 20 or 60 μg/mL. A control group was treated with 0.1% (v/v) dimethyl sulfoxide (DMSO) instead of the sample 1 or 2. Relative NO production was calculated by the following equation.

$$\text{Relative NO production} = (\text{absorbance of sample-treated group} - \text{absorbance of reagent alone}) / (\text{absorbance of control group (DMSO-treated group)} - \text{absorbance of reagent alone})$$

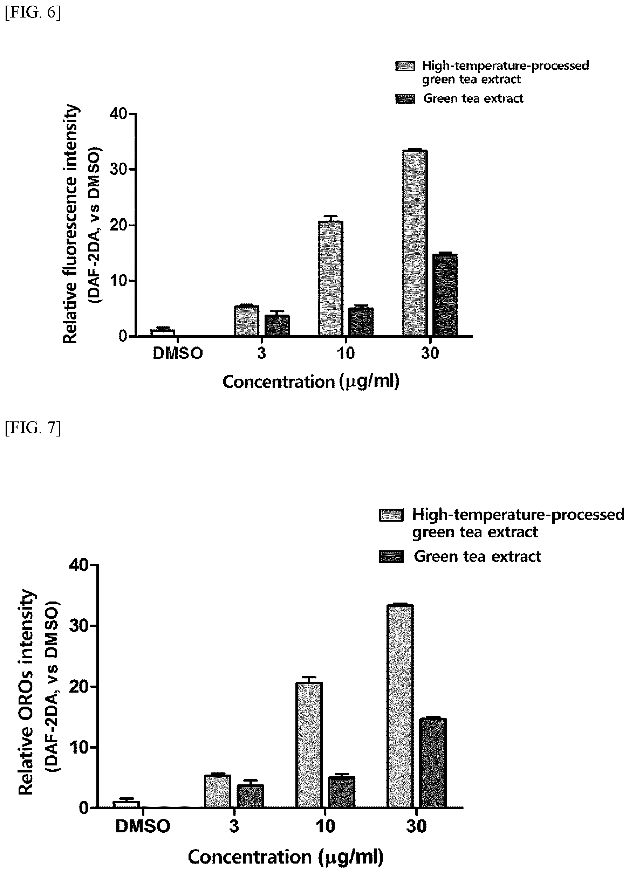

The result is shown in FIG. 6. It was confirmed that, although both the samples 1 and 2 increased NO production in the vascular endothelial cells in a concentration-dependent manner, the high-temperature-processed green tea extract sample 2 according to the present disclosure showed remarkably higher NO production as compared to the common green tea extract sample 1 at the same concentration.

From the results of Test Examples 1 and 2, it can be seen that, when compared with the existing common green tea extract, the high-temperature-processed green tea extract according to the present disclosure exhibits higher safety due to lower hepatotoxicity and exhibits superior blood circulation-enhancing effect by producing a larger amount of NO in vascular endothelial cells at a lower concentration.

**[Test Example 6] Evaluation of effect of inhibiting lipid accumulation in hepatocytes of high-temperature-processed green tea extract**

In order to investigate the effect of inhibiting lipid accumulation in hepatocytes of the green tea extract sample 1 and sample 2 of Example 1, the inhibition of fat accumulation was compared after culturing HepG2 human liver cancer cells and treating with each sample.

Specifically, hepatocytes (HepG2 cells) were seeded onto a 12-well plate (Falcon) with $1 \times 10^6$ cells per well and cultured in a 5% $CO_2$ incubator at 37 °C for 24 hours. Then, after treating with each of the sample 1 and the sample 2 at 3, 10 or 30 μg/mL and 100 μM stearic acid conjugated with bovine serum albumin (BSA), the cells were cultured for 48 hours. After removing the medium and washing with PBS, the cells were fixed with 4% formaldehyde. After incubating with 100% propylene glycol for 5 minutes, the fat in the cells was stained by adding 500 μL of Oil Red O solution (Sigma) per well and culturing the cells for 2 hours. After removing the Oil Red O solution, the Oil Red O solution was extracted from the fat accumulated in the cells by incubating with 85% propylene glycol for 5 minutes and then with 60% isopropanol for 15 minutes. After transferring 200 μL of the isopropanol solution to a 96-well plate, the fat accumulated in the cells was quantified by measuring absorbance at 485 nM. The Oil Red O content (%) relative to a control group (0.1% (v/v) DMSO) was calculated by the following equation.

$$\text{Oil red O content (\%)} = (\text{absorbance of sample-treated group} - \text{absorbance of control group}) \times 100$$

The result is shown in FIG. 7. It was confirmed that the sample 1 resulted in increased fat accumulation at 3 μg/mL and inhibited fat accumulation in a concentration-dependent manner when treated at concentrations of 10 μg/mL and 30 μg/mL. In contrast, the sample 2 according to the present disclosure inhibited fat accumulation in a concentration-dependent manner from the concentration of 3 μg/mL and the fat accumulation-inhibiting ability of the sample 2 was remarkably superior to that of the sample 1.

Taken together with the results of Test Examples 1 and 2, it can be seen that the high-temperature-processed green tea extract according to the present disclosure exhibits superior safety with lower hepatotoxicity as compared to the existing common green tea extract and exhibits superior effect of preventing fatty liver due to excellent effect of inhibiting fat accumulation in hepatocytes.

**[Test Example 7] Evaluation of effect of inhibiting lipid accumulation in hepatocytes of GCG and EGCG**

Experiment was conducted in the same manner as in Test Example 6 except that GCG and EGCG were used instead of the sample 1 and the sample 2.

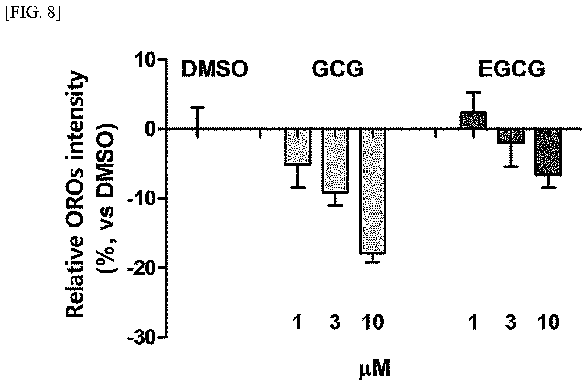

The result is shown in FIG. 8. It was confirmed that GCG exhibits remarkably excellent effect of inhibiting lipid accumulation in hepatocytes as compared to EGCG at the same concentration. Accordingly, it was confirmed that the high-temperature-processed green tea extract according to the present disclosure exhibits superior ability of inhibiting lipid accumulation in hepatocytes because it contains EGCG at a much lower content as compared to the common green tea extract and contains GCG which is not present in the common green tea extract.

**[Test Example 8] Evaluation of effect of inhibiting gingivalis bacteria of high-temperature-processed green tea extract**

In order to investigate the effect of inhibiting gingivalis bacteria of the high-temperature-processed green tea extract, samples were prepared by mixing the ingredients described in Table 5. Each sample was prepared from a 100-fold stock solution and the contents of the ingredients of the sample 2 are the same as those of the eight catechins in Table 3.

[Table 5]

|  | Epicatechin mixture sample | Epicatechin epimer mixture sample | High-temperature-processed green tea extract (sample 2) |
|---|---|---|---|
| EGCG | 421.6 μg/mL | - | 5.27 wt% (206.5 μg/mL) |
| GCG | - | 421.6 μg/mL | 5.59 wt% (219.1 μg/mL) |
| EGC | 337 μg/mL | - | 3.53 wt% (138.3 μg/mL) |
| GC | - | 337 μg/mL | 4.9 wt% (192.0 μg/mL) |
| ECG | 104 μg/mL | - | 1.3 wt% (51.0 μg/mL) |
| CG | - | 104 μg/mL | 1.3 wt% (51.0 μg/mL) |
| EC | 102.8 μg/mL | - | 0.95 wt% (37.2 μg/mL) |
| C | - | 102.8 μg/mL | 1.57 wt% (61.5 μg/mL) |
| Total | 956.6 μg/mL | 956.6 μg/mL | 24.41 wt% (956.6 μg/mL) |

*Porphyromonas gingivalis* was inoculated to brucella agar (Becton Dickison, Le Pont-de-Claix, France) containing vitamin K (1 μg/mL), hemin (5 μg/mL) and 5% defibrinated horse blood and was cultured anaerobically at 37 °C for 40 hours. After collecting the bacteria through centrifugation, the precipitated pellet was washed 3 times with phosphate-buffered saline (PBS). After adding 100 μL of *Porphyromonas gingivalis* to each sample of Table 5 to $2 \times 10^5$ CFU/mL, the bacteria were cultured at 37 °C for at least 16 hours and the minimum inhibitory concentration not showing turbidity was measured. The result is given in Table 6.

[Table 6]

| | Minimum inhibitory concentration (μg/mL) | | |
| --- | --- | --- | --- |
| | Epicatechin mixture sample | Epicatechin epimer mixture sample | High-temperature-processed green tea extract (sample 2) |
| *Porphyromonas gingivalis* | > 8000 | 2000 | 1000 |

From Table 6, it can be seen that the high-temperature-processed green tea extract according to the present disclosure exhibits remarkably superior effect of inhibiting *Porphyromonas gingivalis* as compared to the sample containing four epicatechins or the sample containing four epicatechin epimers. Especially, the sample containing four epicatechins showed very poor effect of inhibiting *Porphyromonas gingivalis,* and the sample containing four epicatechin epimers showed the effect of inhibiting *Porphyromonas gingivalis* but the effect was significantly lower than that of the sample 2 according to the present disclosure.

Taken together with the results of Test Examples 1 and 2, it can be seen that the high-temperature-processed green tea extract according to the present disclosure exhibits high safety with lower hepatotoxicity as compared to the existing common green tea extract and exhibits superior effect of preventing periodontitis due to excellent effect of inhibiting *Porphyromonas gingivalis.*

**[Test Example 9] Evaluation of effect of increasing cerebral blood flow of high-temperature-processed green tea extract**

Near-infrared spectroscopy (NIRS) allows non-invasive monitoring of cerebral blood flow by measuring signals reflecting the degree of oxidation of hemoglobin in blood associated with neural activity.

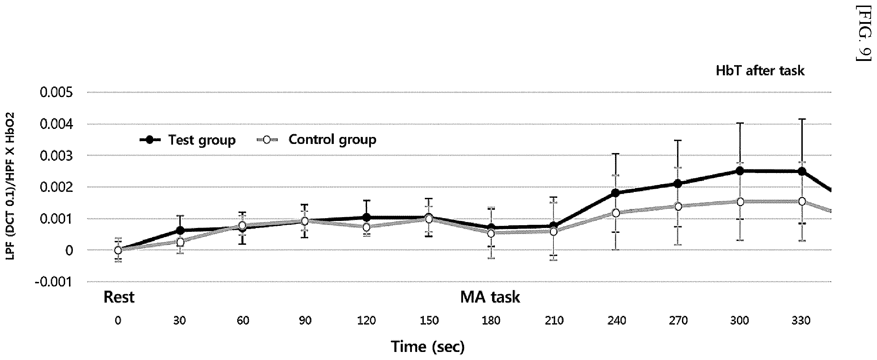

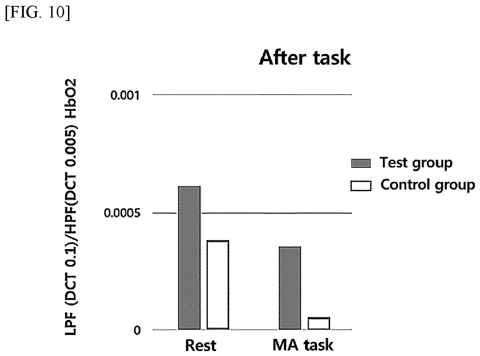

Specifically, the change in cerebral blood flow was monitored by measuring the degree of hemoglobin saturation in the frontal lobe by multichannel near-infrared spectroscopy using a functional near-infrared spectrometer (fNIRS, NIRSIT, OBELAB). First, a test subject was asked to fill out a research consent form and was informed about the progress of the overall experimental procedure. For measurement of basic activity, the subject put on the NIRSIT system and a task was started after 3 minutes' rest. Then, the subject was asked to solve 10 multiplication problems after measurement of the rest state for 30 seconds. After the task was finished, the subject was asked to ingest the high-temperature-processed green tea extract of sample 2 or a control group (450 mg, two tablets) together with enough water. A total of 23 subjects (12 women and 11 men) were randomly assigned to a test group (12 subjects, 6 women and 6 men, $32.17 \pm 1.84$ years) or a control group (11 subjects, 6 women and 5 men, $30.55 \pm 1.26$ years). After waiting for 45 minutes for rest and absorption after the ingestion, the subject put on the NIRSIT system again and a task was started after 3 minutes' rest. After measurement of the rest state (baseline) for 30 seconds, the subject was asked to solve 30 multiplication (math/arithmetic, MA) problems. The result measured by near-infrared spectroscopy was analyzed with a 0.1 Hz low-pass filter (LPF) (DCT 0.1) with or without a 0.005 Hz high-pass filter (HPF) (DCT 0.005). The quantity of total hemoglobin depending on time (FIG. 9) and the quantity of oxyhemoglobin during the task (FIG. 10) were analyzed.

The result is shown in FIG. 9 and 10. It was confirmed that, when the high-temperature-processed green tea extract sample 2 according to the present disclosure was ingested, the quantity of total hemoglobin and oxyhemoglobin in the frontal lobe of the brain was increased remarkably during the MA task, 45 minutes after the ingestion, as compared to the control group.

Taken together with the results of Test Examples 1 and 2, it can be seen that the high-temperature-processed green tea extract according to the present disclosure exhibits high safety with lower hepatotoxicity as compared to the existing common green tea extract and exhibits superior effect of improving cognitive function by increasing blood flow in the frontal lobe of the brain.

**Claims**

**1.** A green tea extract with reduced hepatotoxicity, comprising 8 wt% or less of epigallocatechin (EGC) based on the total weight of the extract.

2. The green tea extract according to claim 1, wherein the extract comprises 4-15 wt% of (-)-gallocatechin gallate (GCG) and 4-15 wt% of (-)-epigallocatechin gallate (EGCG) based on the total weight of the extract.

3. The green tea extract according to claim 2, wherein the total content of (-)-epigallocatechin gallate (EGCG), epigallocatechin (EGC), (-)epicatechin (EC), epicatechin 3-O-gallate (ECG), gallocatechin gallate (GCG), gallocatechin (GC), catechin (C) and catechin gallate (CG) in the extract is 19-30 wt% based on the total weight of the extract.

4. The green tea extract according to claim 1, wherein the extract is an extract obtained by extracting one or more times with one or more of water and a $C_1$-$C_4$ alcohol.

5. A composition comprising the green tea extract according to any of claims 1 to 4 as an active ingredient.

6. The composition according to claim 5, wherein the green tea extract is comprised in an amount of 1-100 wt% based on the total weight of the composition.

7. The composition according to claim 5, wherein the composition is a functional health food or pharmaceutical composition.

8. The composition according to claim 5, wherein the composition is for reducing hepatotoxicity.

9. The composition according to claim 5, wherein the composition is for enhancing blood circulation.

10. The composition according to claim 9, wherein the composition is for increasing nitric oxide (NO) in vascular endothelial cells.

11. The composition according to claim 5, wherein the composition is for inhibiting lipid accumulation in hepatocytes.

12. The composition according to claim 5, wherein the composition is for inhibiting gingivalis bacteria.

13. The composition according to claim 12, wherein the gingivalis bacterium is *Porphyromonas gingivalis.*

14. The composition according to claim 5, wherein the composition is for increasing cerebral blood flow.

[FIG. 1]

[FIG. 2]

[FIG. 3]

[FIG. 4]

[FIG. 5]

[FIG. 6]

[FIG. 7]

[FIG. 8]

[FIG. 9]

[FIG. 10]

## INTERNATIONAL SEARCH REPORT

| | International application No. |
|---|---|
| | **PCT/KR2019/014810** |

| A. CLASSIFICATION OF SUBJECT MATTER |
|---|
| *A61K 36/82(2006.01)i, A23L 33/00(2016.01)i, A23L 5/20(2016.01)i* |
| According to International Patent Classification (IPC) or to both national classification and IPC |

| B. FIELDS SEARCHED |
|---|
| Minimum documentation searched (classification system followed by classification symbols) |
| A61K 36/82; A23F 3/16; A23L 1/164; A23L 1/30; A61K 8/97; A61P 3/04; A23L 33/00; A23L 5/20 |
| Documentation searched other than minimum documentation to the extent that such documents are included in the fields searched |
| Korean utility models and applications for utility models: IPC as above |
| Japanese utility models and applications for utility models: IPC as above |
| Electronic data base consulted during the international search (name of data base and, where practicable, search terms used) |
| eKOMPASS (KIPO internal) & Keywords: green tea extract, epigallocatechin, reducing hepatotoxicity |

| C. DOCUMENTS CONSIDERED TO BE RELEVANT | | |
|---|---|---|
| Category* | Citation of document, with indication, where appropriate, of the relevant passages | Relevant to claim No. |
| X | KR 10-2005-0022250 A (SAM-A PHARM. CO., LTD.) 07 March 2005<br>See abstract; claims 1-7. | 1-8,11 |
| X | KR 10-2010-0124519 A (AMOREPACIFIC CORPORATION) 29 November 2010<br>See abstract; claims 1-20. | 1-7,9-10,14 |
| X | ZHAO, L. et al. Antibacterial, antiadherence, antiprotease, and anti-inflammatory activities of various tea extracts: potential benefits for periodontal diseases. J Med Food. 2013, vol. 16, no. 5, pages 428-436<br>See abstract; figure 1. | 1-7,12-13 |
| A | KR 10-2015-0016343 A (DSM NUTRITIONAL PRODUCTS AG.) 11 February 2015<br>See paragraph [0068]; and claims 1, 32. | 1-14 |
| A | KIM, Min Chae et al. Robust optimization for the simultaneous enhancement of nitric oxide inhibition and reduction of hepatotoxicity from green tea catechins. Food Sci Biotechnol. 2017, vol. 26, no. 6, pages 1725-1734<br>See abstract; figure 2. | 1-14 |
| A | WO 2015-199169 A1 (KYUSHU UNIVERSITY, NATIONAL UNIVERSITY CORPORATION) 30 December 2015<br>See claims 1, 5. | 1-14 |

| ☒ Further documents are listed in the continuation of Box C. | ☒ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 29 JANUARY 2020 (29.01.2020) | **29 JANUARY 2020 (29.01.2020)** |

| Name and mailing address of the ISA/KR | Authorized officer |
|---|---|
| Korean Intellectual Property Office<br>Government Complex Daejeon Building 4, 189, Cheongsa-ro, Seo-gu, Daejeon, 35208, Republic of Korea<br>Facsimile No. +82-42-481-8578 | Telephone No. |

Form PCT/ISA/210 (second sheet) (January 2015)

**INTERNATIONAL SEARCH REPORT**

International application No.

**PCT/KR2019/014810**

C (Continuation). DOCUMENTS CONSIDERED TO BE RELEVANT

| Category* | Citation of document, with indication, where appropriate, of the relevant passages | Relevant to claim No. |
|---|---|---|
| A | KR 10-2003-0009578 A (AMOREPACIFIC CORPORATION) 05 February 2003<br>See experimental example 1; claim 1; and table 1. | 1-14 |

Form PCT/ISA/210 (continuation of second sheet) (January 2015)

INTERNATIONAL SEARCH REPORT
Information on patent family members

International application No.

**PCT/KR2019/014810**

| Patent document cited in search report | Publication date | Patent family member | Publication date |
|---|---|---|---|
| KR 10-2005-0022250 A | 07/03/2005 | KR 10-0592792 B1 | 26/06/2006 |
| KR 10-2010-0124519 A | 29/11/2010 | CN 102438642 A | 02/05/2012 |
| | | JP 2012-527450 A | 08/11/2012 |
| | | JP 6030447 B2 | 24/11/2016 |
| | | US 2012-0052138 A1 | 01/03/2012 |
| | | WO 2010-134756 A2 | 25/11/2010 |
| | | WO 2010-134756 A3 | 10/03/2011 |
| | | WO 2010-134756 A8 | 25/11/2010 |
| KR 10-2015-0016343 A | 11/02/2015 | CA 2874379 A1 | 28/11/2013 |
| | | CA 2874379 C | 10/09/2019 |
| | | CN 104602538 A | 06/05/2015 |
| | | CN 110074333 A | 02/08/2019 |
| | | EP 2852294 A1 | 01/04/2015 |
| | | EP 2852294 B1 | 19/10/2016 |
| | | JP 2015-517318 A | 22/06/2015 |
| | | JP 6376499 B2 | 22/08/2018 |
| | | KR 10-1983871 B1 | 29/05/2019 |
| | | WO 2013-175253 A1 | 28/11/2013 |
| WO 2015-199169 A1 | 30/12/2015 | US 10251408 B2 | 09/04/2019 |
| | | US 2017-0156361 A1 | 08/06/2017 |
| KR 10-2003-0009578 A | 05/02/2003 | KR 10-0637653 B1 | 24/10/2006 |

Form PCT/ISA/210 (patent family annex) (January 2015)

## REFERENCES CITED IN THE DESCRIPTION

**Patent documents cited in the description**

- KR 1020180134620
- KR 1020190037810
- KR 1020190129034